# EUROPEAN PATENT SPECIFICATION

(11) **EP 2 173 255 B1**
(45) Date of publication and mention of the grant of the patent: **18.04.2012**
(21) Application number: 08755419.2
(22) Date of filing: 14.05.2008
(51) Int. Cl.: A61B 17/00

(54) **MINIMALLY INVASIVE SURGICAL TOOLS WITH HAPTIC FEEDBACK**
MINIMAL INVASIVE OPERATIONSINSTRUMENTE MIT HAPTIK-FEEDBACK
OUTILS CHIRURGICAUX À INVASIVITÉ MINIMALE DOTÉS D'UN RETOUR HAPTIQUE

(30) Priority: 09.07.2007 US 948616 P; 13.12.2007 US 955563
(43) Date of publication of application: 14.04.2010
(73) Proprietor: Immersion Corporation, San Jose, CA 95131 (US)
(72) Inventor: RAMSTEIN, Christophe, San Francisco, CA 94133 (US); ULLRICH, Christopher, J., Sana Cruz, CA 95060 (US); DEGHEEST, Anne, Los Altos Hills, CA 94022 (US)
(74) Representative: Hofstetter, Alfons J.
(86) International application number: PCT/US2008/063561
(87) International publication number: WO 2009/009220

(56) References cited:
- WO-A-03/020139
- WO-A-2004/067053
- WO-A-2005/013803
- CA-A- 2 520 942
- DE-A- 4 213 426
- US-A1- 2001 025 150
- US-A1- 2002 120 188
- US-A1- 2004 167 559

## Description

The invention is directed to surgical tools as described in the preamble of claim 1.

Minimally invasive surgery is performed without making a major incision or opening, resulting in reduced trauma for the patient and yielding significant cost savings. These result from shorter hospitalization times and reduced therapy requirements. Other benefits of minimally invasive surgery include less pain, less need for post-surgical pain medication, less scarring, and less likelihood of complications related to the incision.

Minimally invasive surgery is defined either as based on the operative procedure (e.g., small incisions) or the outcome (e.g., reduced surgical complications or costs). However, minimally invasive surgery is not the same as minor surgery. Some "minimally invasive" procedures, e.g., coronary artery bypass surgery, still are major operations requiring a hospital stay.

In minimally invasive surgery, a miniature camera is typically introduced into the body through a small incision. The camera transmits images to a video monitor, enabling the physician to diagnose and, if necessary, treat a variety of conditions. To do this, the physician inserts surgical instruments and auxiliary devices (collectively, "minimally invasive surgical tools"), such as irrigation and drainage devices, through one or more additional small incisions. Such surgical instruments can be for laparoscopic surgery, catheterization or endoscopy, as well as for enabling telesurgery and telepresence. Compared to open surgery, however, minimally invasive surgery presents limitations in visual and haptic perceptions, and creates challenges unique to this type of surgery. One of the major concerns is the potential for tissue damage, possibly caused by inappropriate use of force.

WO-A-03020139, on which the preamble of claim 1 is based, discloses an instrument for minimally invasive surgery comprising means for feeding back a force which is exerted on the working element of the instrument to the operating element. These means comprise at least a first force sensor for measuring the force which is exerted on the working element, a control unit and a first actuator. US-A-2001025150 discloses surgical devices that provide perceptual feedback to a medical practitioner in the form of e.g. tactile sensations or auditory feedback, and methods of use of the devices. CA-A-2520942 discloses a tool comprising a handle and a tip, which provides amplified tactile feedback to a user regarding the material with which the tip of the tool is in contact.

Based on the foregoing, there is a need for improved minimally invasive surgical tools.

### SUMMARY OF THE INVENTION

The invention discloses a minimally invasive surgical tool comprising the features of claim 1.

### BRIEF DESCRIPTION OF THE DRAWINGS

Fig. 1 illustrates a minimally invasive surgical tool.

Fig. 2 is a block diagram of a haptic feedback system.

Fig. 3 illustrates a minimally invasive surgical tool in accordance with one embodiment.

Fig. 4 illustrates a minimally invasive surgical tool and corresponding trocar.

### DETAILED DESCRIPTION

One embodiment is a minimally invasive surgical tool that includes a sensor near or at the tool tip and that generates haptic effects to provide relevant feedback to a user that is operating the tool.

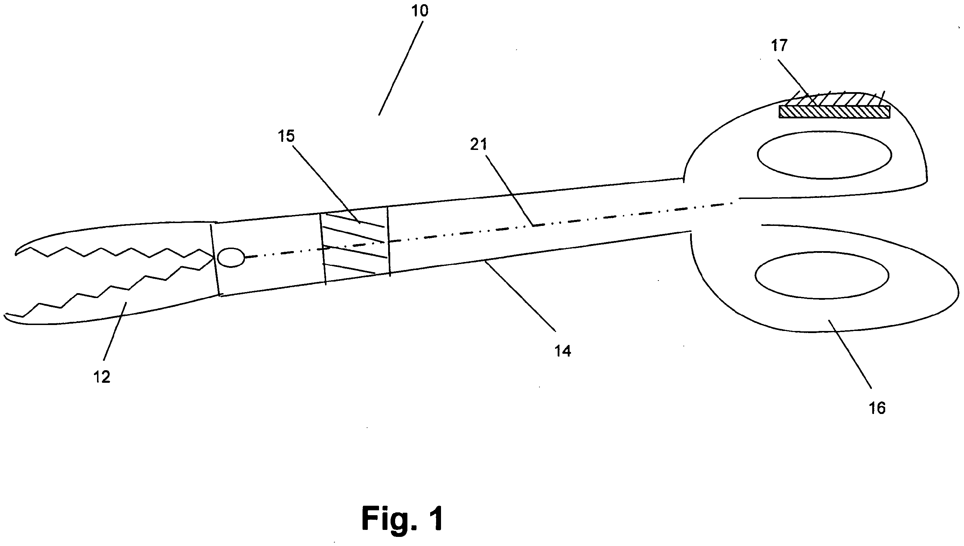

Fig. 1 illustrates a minimally invasive surgical tool 10 in accordance with one embodiment. Tool 10 is a gripping tool for laparoscopy surgery, but may be any type of minimally invasive surgical tool, including a tool that is specifically designed to provide haptic feedback through interaction with a body. Tool 10 includes a tool tip, which in the embodiment shown is a gripper portion 12, a housing 14 and a handle 16. The motion of handle 16 opens and closes gripper portion 12 through an internal wire or tendon 21 that runs from handle 16 to gripper portion 12. Laparoscopic tools in general, like tool 10, are typically 5mm-10mm thin instruments that each have varied functions (e.g., grippers, scissors, clip appliers, etc.) and that can be introduced by the surgeon into the abdomen or other areas of the body through trocars, which are hollow tubes with a rubber seal to keep CO₂ from leaking.

When using a prior art gripping tool, the surgeon will typically poke at or otherwise interact with tissue with gripper portion 12, and rely on a muted feeling at handle 16 in order to determine the stiffness of the tissue. However, this technique is not very accurate and can lead to potentially fatal mistakes when the wrong tissue is cut or gripped. Housing 14 includes a flexible and encapsulating strain gauge 15. Strain gauge 15 is installed by removing a portion of housing 14 and replacing with gauge 15. In one embodiment, strain gauge 15 is installed near the tip of tool 10.

Tool 10 further includes a haptic feedback system 17 coupled to handle 16 or some other portion of tool 10 so that the user contacts a portion of haptic feedback system 17 when performing a procedure. In one embodiment, haptic feedback system 17 is a vibrotactile device that generates vibrations for haptic feedback. In other embodiments, other types of haptic feedback are generated and provided to the user, such as kinesthetic feedback (e.g., active and resistive force feedback) and/or other types of tactile feedback besides vibration (e.g., texture and heat). Haptic feedback system 17 is coupled to strain gauge 15 internally to tool 10 in one embodiment.

In operation, signals are generated by strain gauge 15 as the tip of tool 10 (e.g., gripper portion 12) interacts with the bone and various types of tissue found in a human or other animal body and creates deformation in gauge 15. The signals received from gauge 15 may be "amplified" by being converted into corresponding haptic feedback so that the user performing the operation has an "enhanced" feel for the tissue and bone that he/she is navigating through and around. This enhanced feel, a magnification of the forces applied to the surgical tip of the device during use (i.e., cutting, catheterization, etc.) provides the user with better control and sensitivity for using the device effectively, efficiently and with minimal trauma to the patient. Further, during palpation, force sensed at the tool tip is translated into haptic feedback, either to amplify or highlight the internal interaction of the tool tip with the body.

The haptic feedback in one embodiment may be vibrotactile that is varied or "dynamic" based on a changing level of stiffness or deformation. The variation may be a change of amplitude, frequency, duration, etc. Other types of haptic feedback may include kinesthetic feedback using solenoids to change the stiffness/damping of handle 16, small air bags that change size in handle 16, or shape changing materials. In another embodiment, a user may wear force feedback cyber gloves that include multiple force output capabilities. All embodiments may include combinations of different types of haptic feedback, or combinations of haptic feedback and non-haptic feedback (e.g., audio/visual feedback).

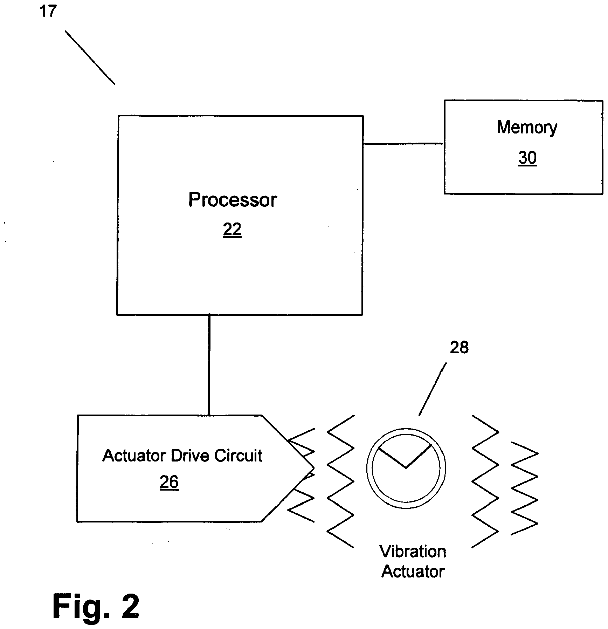

Fig. 2 is a block diagram of haptic feedback system 17. Haptic feedback system 17 includes a processor 22 coupled to a memory 30 and an actuator drive circuit 26 which is coupled to a vibration actuator 28. Processor 22 may be any type of general purpose processor, or could be a processor specifically designed to provide haptic effects, such as an application-specific integrated circuit ("ASIC"). Processor 22 can determine what haptic effects are to be played and the order in which the effects are played based on high level parameters and in response to signals received from strain gauge 15. In general, the high level parameters that define a particular haptic effect include magnitude, frequency and duration. Low level parameters such as streaming motor commands could also be used to determine a particular haptic effect.

Processor 22 outputs control signals to drive circuit 26 which includes electronic components and circuitry used to supply actuator 28 with the required electrical current and voltage to cause the desired haptic effects. Actuator 28 is a haptic device that generates a vibration on handle 16. Actuator 28 can include one or more force applying mechanisms which are capable of applying a vibrotactile force to a user of device 10. Actuator 28 may be, for example, an electromagnetic actuator, an Eccentric Rotating Mass ("ERM") in which an eccentric mass is moved by a motor, a Linear Resonant Actuator ("LRA") in which a mass attached to a spring is driven back and forth, or a "smart material" such as piezoelectric, electro-active polymers or shape memory alloys.

Memory device 30 can be any type of storage device or computer-readable medium, such as random access memory ("RAM") or read-only memory ("ROM"). Memory 30 stores instructions executed by processor 22. Memory 30 may also be located internal to processor 22, or any combination of internal and external memory.

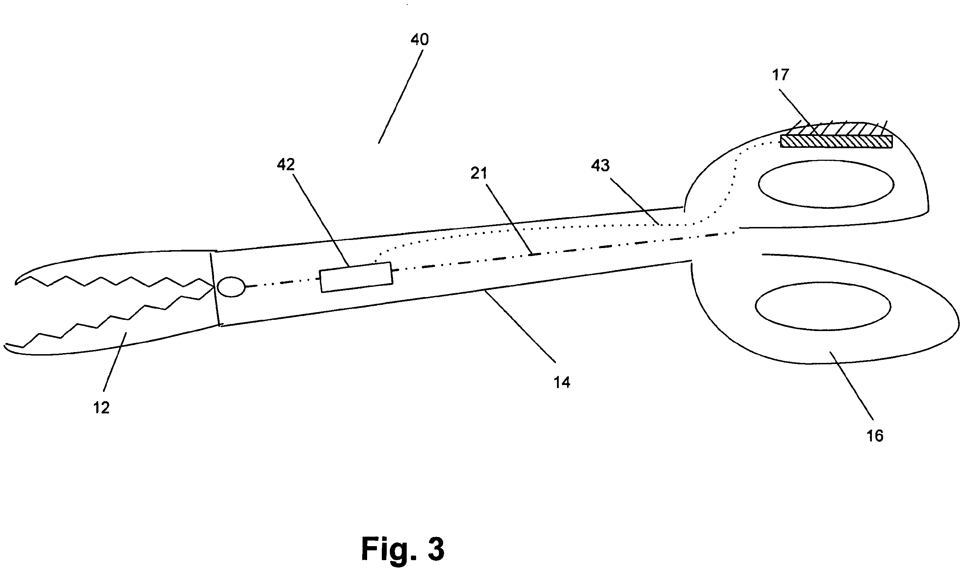

Fig. 3 illustrates a minimally invasive surgical tool 40 in accordance with one embodiment. In tool 40, a portion of tendon 21 is replaced with an in-line piezo strain gauge 42 along its length. Gauge 42 outputs a signal that is proportional to how hard handle 16 is being squeezed, which is reflective on how hard grippers 12 are gripping. This enables the signal to indicate, for example, whether grippers 12 are contacting bone, tissue or a blood vessel, or even whether a contacted blood vessel is pulsating. Therefore, the user can more easily probe using tool 40. The signal from gauge 42 is transmitted to haptic system 17 via line 43, where it is converted to a haptic feedback that can be detected by the user.

In embodiments not according to the invention, the interaction between the tool tip and the body may not be a physical force (e.g., the tip contacting a bone) but some other type of interaction. Other examples of interaction can be implemented by coupling a sensor on the tool tip. In one embodiment, a piezoelectric transducer that senses acoustic vibration is mounted on the tool tip, and the interaction is the sensing of acoustic vibrations by the transducer, which in turn generates the signal that is received by the haptic feedback system. Additional examples of sensors that can be coupled to the tool tip include a pressure transducer, a silicon chip that is sensitive to biological materials, an ultrasound probe, an electro-magnetic field sensor, etc.

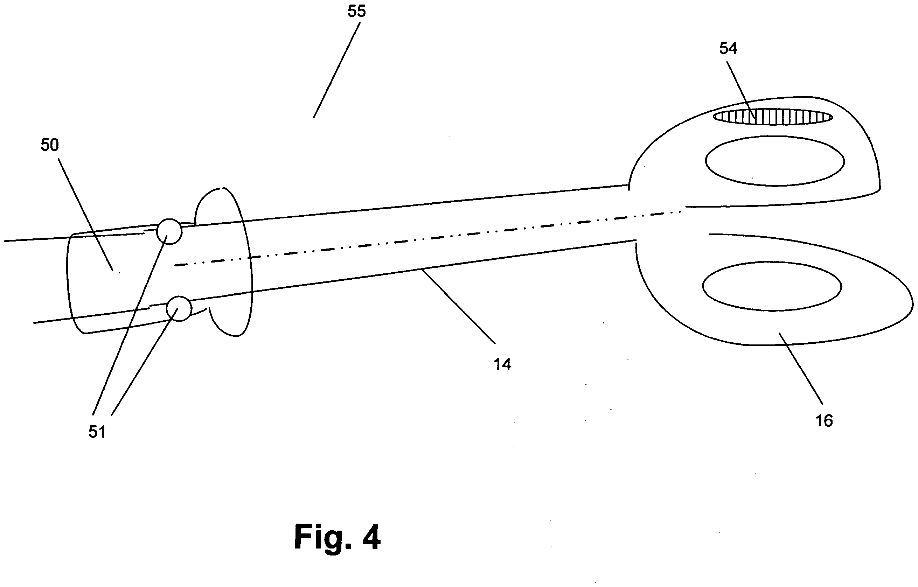

Fig. 4 illustrates a minimally invasive surgical tool 55 and corresponding trocar 50. Trocar 50 includes rollers 51 around its inner circumference, rather than a passive rubber seal as in prior art trocars. The prior art rubber seal does not provide varied friction, and filters out much of the feedback or feeling that a user might receive at handle 16. Rollers 51 include actuators or other structure that allow the rolling resistance of rollers 51 on the outside of housing 14 to be varied. A wire or other interface couples the actuators to a pressure sensor 54 located on handle 16. The user can vary the amount of resistance applied by rollers 51 by varying the pressure applied to pressure sensor 54. For example, the tighter a user grips handle 16, the greater resistance may be applied by rollers 51.

Trocar 50 can be utilized in multiple ways to enhance the use of tool 55. For example, the resistance of rollers 51 may be maximized so housing 14 can be "stuck" in place within trocar 50. Further, the resistance of rollers 51 may be minimized so that housing 14 slides effortlessly and nearly friction-free through trocar 50, thus enhancing the "feedback" provide to the user at handle 16 when probing. In one embodiment, trocar 50 is considered part of tool 55.

In one embodiment, the sensors and/or actuators in the tool are disposable items. Typically, in vivo devices such as laparoscopy tools are required to be sterilized before each use. Removable, disposable portions of the tool would not have to be sterilized because they would just be replaced. In one embodiment, the handles with the actuators can be screwed off and replaced. In other embodiments, the sensor may also be disposable.

For example, although embodiments disclosed are tools for laparoscopic surgery, other embodiments can be used for non-laparoscopic surgeries such as in catheterization where ultrasonic imaging or other mechanical sensors on the tool-tip can be communicated back to the catheter handle. Further, for endoscopy procedures, mechanical sensors on a flexible endoscope can communicate local tissue properties such as mechanical impedance. Other embodiments can be used for telesurgery or telepresence in order to, for example, perform routine external examinations by a remote doctor.

## Claims

1. A minimally invasive surgical tool (40) comprising:
a sensor that generates a signal in response to an interaction with the tool (40);
an internal wire (21); and
a haptic feedback system (17) that generates a haptic effect in response to the signal, wherein
said signal is proportional to how hard a handle (16) of the tool (40) is being squeezed, which is reflective on how hard grippers (12) of the tool (40) are gripping a material therebetween,
**characterized in that**
the sensor is an inline piezo strain gauge (42), and the strain gauge (42) is coupled to the internal wire (21), wherein a portion of the internal wire (21) is replaced with said strain gauge (42) along its length.

2. The tool of claim 1, wherein the haptic feedback system comprises an actuator (28), and the haptic effect is vibrotactile.

3. The tool of claim 1, wherein the haptic effect is dynamic.

## Patentansprüche

1. Minimal invasives chirurgisches Instrument (40), das umfasst:
einen Sensor, der ein Signal in Reaktion auf eine Wechselwirkung mit dem Instrument (40) erzeugt;
einen internen Draht (21); und
ein haptisches Rückkopplungssystem (17), das einen haptischen Effekt in Reaktion auf das Signal erzeugt, wobei das Signal dazu proportional ist, wie hart ein Griff (16) des Instruments (40) gepresst wird, was widerspiegelt, wie hart Greifer (12) des Instruments (40) ein Material dazwischen greifen,
**dadurch gekennzeichnet, dass**
der Sensor ein Inline-Piezo-Dehnungsmesser (42) ist und der Dehnungsmesser (42) mit dem internen Draht (21) gekoppelt ist, wobei ein Abschnitt des internen Drahts (21) durch den Dehnungsmesser (42) entlang seiner Länge ersetzt ist.

2. Instrument nach Anspruch 1, wobei das haptische Rückkopplungssystem einen Aktuator (28) umfasst und der haptische Effekt vibrotaktil ist.

3. Instrument nach Anspruch 1, wobei der haptische Effekt dynamisch ist.

## Revendications

1. Instrument chirurgical mini-invasif (40), comprenant :
un capteur qui génère un signal en réponse à une interaction avec l'instrument (40) ;
un fil métallique interne (21) et
un système de réaction haptique (17), qui génère un effet haptique en réponse au signal, dans lequel ledit signal est proportionnel à la dureté, avec laquelle une poignée (16) de l'instrument (40) est pressée, laquelle reflète la dureté avec laquelle les pinces (12) de l'instrument (40) pincent un matériau entre elles,
**caractérisé en ce que**
le capteur est une jauge de contrainte piézoélectrique en ligne (42) et la jauge de contrainte (42) est couplée au fil métallique interne (21), dans lequel une partie du fil métallique interne (21) est remplacée par ladite jauge de contrainte (42) sur sa longueur.

2. Instrument suivant la revendication 1, dans lequel le système de réaction haptique comprend un actionneur (28) et l'effet haptique est vibrotactile.

3. Instrument suivant la revendication 1, dans lequel l'effet haptique est dynamique.
